# EUROPEAN PATENT APPLICATION

(11) **EP 2 172 859 A1**
(43) Date of publication of application: **07.04.2010**
(21) Application number: 08165740.5
(22) Date of filing: 02.10.2008
(51) Int. Cl.: G06F 19/00, A61B 5/00, H04L 12/00, H04N 7/15

(54) **Home health system**

(71) Applicant: EMedLink ApS, 2750 Ballerup (DK)
(72) Inventor: Rosager, Marianne, 3500 Værløse (DK)
(74) Representative: Inspicos A/S

(57) **Abstract**

A home health system comprises a home system comprising a medical device for capturing health data related to a health condition of a patient. The home system is configured to pass the health data to a healthcare facility system via a wide area network. The home health system further includes videoconferencing equipment configured to enable videoconferencing between the patient and medical staff at the medical health care facility via the wide area network. All data and videoconference sequences are passed through a server system at a site remote from the home system and remote from the healthcare facility system, where the data and videoconference sequences are stored and docketed. The home health system is particularly useful in monitoring and attending to patients suffering from a chronic disease, such as chronic obstructive pulmonary disease (COPD).

## Description

### Technical field

The present invention relates to a home health system for use in telemedicine. Home health systems are useful for attending to patients at locations remote from medical healthcare facilities, such as in the patients' homes. The invention also relates to an interface terminal for use in such a system, to a method of monitoring a health condition of a patient, to a method of attending to a patient, and to a method of docketing health data.

### Background of the invention

In recent years, the demographic development in the industrialized world has developed towards an age distribution in the population, which includes a still increasing proportion of elderly persons. Further, the occurrence of chronic diseases is on the rise.

One chronic disease, which is common among elderly persons, is the chronic obstructive pulmonary disease. Chronic obstructive pulmonary disease (COPD) is a disease of the lungs in which the airways become narrowed. This leads to a limitation of the flow of air to and from the lungs causing shortness of breath. In contrast to asthma, the limitation of airflow is poorly reversible and usually gradually gets worse over time. COPD is caused by noxious particles or gases, most commonly from smoking, which trigger an abnormal inflammatory response in the lung. The inflammatory response in the larger airways is known as chronic bronchitis. The natural course of COPD is characterized by occasional sudden worsenings of symptoms called acute exacerbations, most of which are caused by infections or air pollution. The diagnosis and monitoring of COPD requires lung function tests, and COPD patients are often hospitalized on regular intervals for 1 to 2 weeks.

Other chronic diseases, which put great demands on healthcare systems are diabetes, aids, and cancer. Further, a number of non-chronic diseases and conditions are burdensome on healthcare systems.

As a result of the increasing proportion of elderly persons in society and the increasing occurrence of chronic diseases, public and private healthcare costs are steadily increasing. The reduction of healthcare costs consequently has high priority, not only with regard to chronic diseases, but also with regard to non-chronic diseases, acute conditions and emergencies. In order to cut down on healthcare costs, home health systems have been proposed with the aim of enabling patients to undergo certain medical procedures in their private homes or at other facilities remote from hospitals or other medical healthcare facilities. Telemedicine systems have been proposed in order to provide electronic health care and information services to patients.

However, the known home health care systems suffer from various disadvantages, as they are difficult to operate, in particular to elderly patients with reduced dexterity and limited computer user skills. A further disadvantage of the known systems is that they are complex and their installation is expensive. Moreover, many of the known systems have been developed as emergency systems, which are not suitable for monitoring, attending to and treating chronically ill patients. A further shortcoming of the known systems is that docketing of patient health data is often not considered, whereby an additional burden is put on the medical staff in recording and documenting medical procedures or the progress of a disease, thus obstructing a wide acceptance of home health systems in the medical society.

### Summary of the invention

It is hence an object of embodiments of the invention to provide a home health system, which is easy to operate, and which meets the limitations and needs of elderly persons with reduced dexterity and computer skills. It is a further object of embodiments of the invention to provide a system, the installation of which is cost efficient and hence attractive in public healthcare systems. It is a still further object of the invention to provide a system, which is suitable in the surveillance, treatment and docketing of chronic diseases as well as non-chronic diseases.

In one embodiment, the invention provides a home health system comprising:
**(a)** a home system comprising at least one medical device for capturing health data related to a health condition of a patient, the home system further comprising a data interface device configured to receive the health data from the at least one medical device and to pass the health data into a wide area network;
**(b)** a healthcare facility system at a site remote from the home system, the healthcare facility system being configured to receive said health data communicated from the data interface device of the home system via the wide area network;
**(c)** videoconferencing equipment connected to or integrated with the home system and the healthcare facility system, the videoconferencing being configured to enable videoconferencing between the patient and medical staff at the medical health care facility via the wide area network;
**(d)** a server system at a site remote from the home system and remote from the healthcare facility system;
wherein the home system, the health care facility system, the videoconferencing equipment and the server system are configured such that all video data and health care data communicated between the home system and the healthcare facility system are passed through the server system.

The videoconferencing equipment provides a convenient and safe way of exchanging information between the patient and medical staff, and it provides a sense of comfort to elderly patients. Moreover, videoconferencing allows the medical staff to thoroughly interview the patient and to enhance the staffs impression of the patient's condition. The server system provides a number of benefits, as it may be used to control and monitor traffic, and provide protection against unauthorized data access. Additionally, the server system may include database structure allowing storing and docketing of health data, thereby alleviating medical staff from the task of keeping track of the health data and additional information provided during videoconference sessions.

The medical device for capturing health data may e.g. include medical measurement equipment, such as a lung capacity meter or lung airflow meter, ECG or EEG equipment, heart rate meter, body temperature thermometer, blood glucose meter, etc. The data interface for receiving the health data and passing them into the wide area network may include a data interface device according to the present invention, preferably a hand-held device, or a suitably programmed computer, PDA or the like. The data interface device, computer, PDA or the like may include a data input port for receiving the health data from the medical device, e.g. a wire input port or a wireless port for receiving radio communication, Bluetooth signals or inductive signals, suitably configured to receive data transmitted by the medical device.

The healthcare facility system preferably comprises a computer, PDA or the like, by means of which the physician may log on to the server system and/or link to the patient.

In a further embodiment, the invention provides a data interface device, which is preferably hand held, for use in the home health system according to the invention, comprising:
- data input structure for receiving health data communicated by an external device;
- a network interface for connecting the data interface device to a wide area network;
- audio and video structure configured to enable videoconferencing by means of the data interface device, the audio and video structure including a monitor, a loudspeaker, and a microphone integrated in a housing of the data interface device;
- a user interface configured to receive a user input, the user input being limited to pre-defined response options to pre-programmed or pre-packed questions passed to the user.

The hand-held device is preferably accessible for programming thereof via the wide area network. By allowing the user interface of the hand-held device to communicate only a pre-programmed or pre-packed questions to the user, the memory, processor and programming requirements for the hand-held device may be kept at a minimum, and hence maintenance of a great number of hand-held devices at patients' homes is greatly facilitated. Moreover, reconfiguration of the hand-held device upon transfer of the device from one patient to another may be carried out swiftly and easily via the wide area network with a minimum of programming or data loading being required.

In a further embodiment, the invention provides a method of monitoring a health condition of a patient, such as a chronically ill patient, comprising the steps of:
- providing a home system in the patient's home, the home system comprising at least one medical device for capturing health data related to said health condition;
- passing the health data into a wide area network;
- providing a healthcare facility system at a site remote from the home system, and receiving said health data at the healthcare facility via the wide area network;
- enabling a videoconference between the patient and medical staff at the medical health care facility via the wide area network;
- providing a server system at a site remote from the home system and remote from the healthcare facility system;
the method further comprising the step of passing all video data and health care data communicated between the home system and the healthcare facility system through the server system.

In a still further embodiment, the invention provides a method of attending to a patient, such as chronically ill patient at a location remote from a medical healthcare facility, such as the patient's home, comprising:
- causing the patient to capture health data related to a health condition of the patient by means of a medical device;
- passing the health data into a wide area network;
- providing a healthcare facility system at a site remote from the home system, and receiving said health at the healthcare facility data via the wide area network;
- establishing a videoconference between the patient and medical staff at the medical health care facility via the wide area network;
- providing a server system at a site remote from the home system and remote from the healthcare facility system;
the method further comprising the step of passing all video data and health care data communicated between the home system and the healthcare facility system through the server system.

The methods of monitoring the health condition of a chronically ill patient and of attending to such a patient provide the possibility of 'home hospitalization' of chronically ill patients, thereby increasing the patients' as well as their relatives' quality of life, and significantly reducing hospitalization costs and hospital resource requirements.

In a still further embodiment, the invention provides a method of docketing health data of a patient, the method comprising the steps of:
- providing a home system in the patient's home, the home system comprising at least one medical device for capturing health data related to said health condition;
- passing the health data into a wide area network;
- providing a healthcare facility system at a site remote from the home system, and receiving said health data at the healthcare facility via the wide area network;
- providing a server system at a site remote from the home system and remote from the healthcare facility system;
the method further comprising the step of passing the health care data communicated between the home system and the healthcare facility system through the server system, and storing and docketing said data in a database of the server system.

Thanks to the step of passing health care data through the server system, the server system and its database structure or other data-storing structure allows for storing and docketing of health data, thereby alleviating medical staff from the task of keeping track of the health data.

It will be appreciated that the methods of the present invention may be carried out in embodiments of the home health system of the invention and may be partially carried out with the aid of the data interface device according to the invention.

### Description of the drawings

Fig. 1 is a block diagram of an embodiment of a home health system according to the present invention;
Fig. 2 is a block diagram of an embodiment of a home system of the home health system of Fig. 1;
Fig. 3 is a block diagram of an embodiment of a server system of the home health system of Fig. 1.

### Detailed description of embodiments of the invention

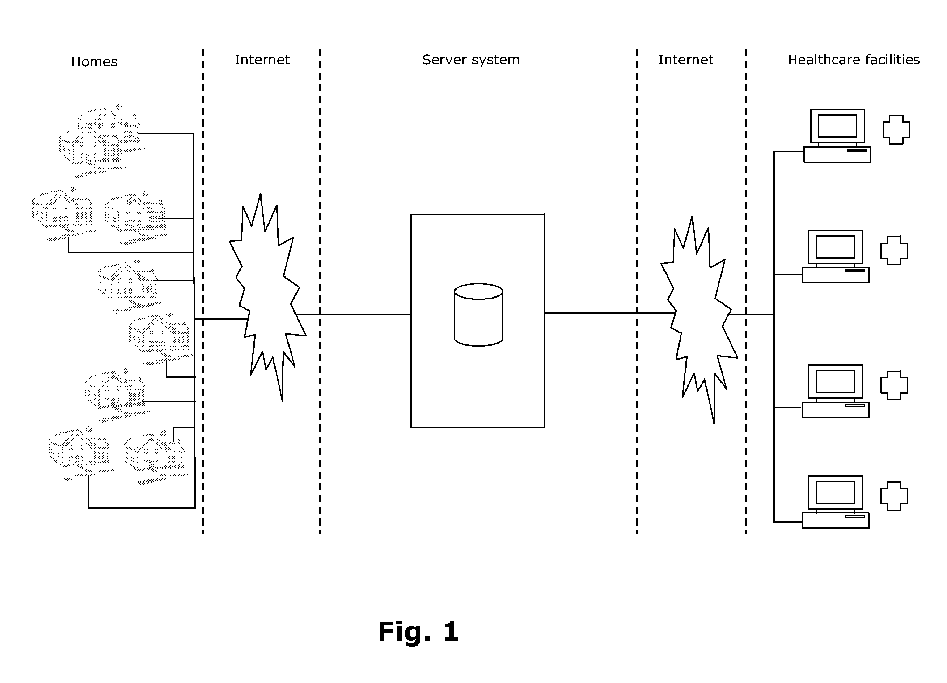

Fig. 1. shows an embodiment of a home health system according to the present invention. A server system as discussed in more detail below includes a file/data storage and is connected to a plurality of healthcare facilities, such as hospitals via a wide area network, such as the Internet. The wide area network also connects the server system to a plurality of homes, each of which includes a home system as disclosed in further detail below. Each home may be allowed to connect only to a specific healthcare facility, or to a number of healthcare facilities. The communication between the home systems and the server system as well as between the server system and the healthcare facilities is preferably encrypted to protect the transmitted data against unauthorized access.

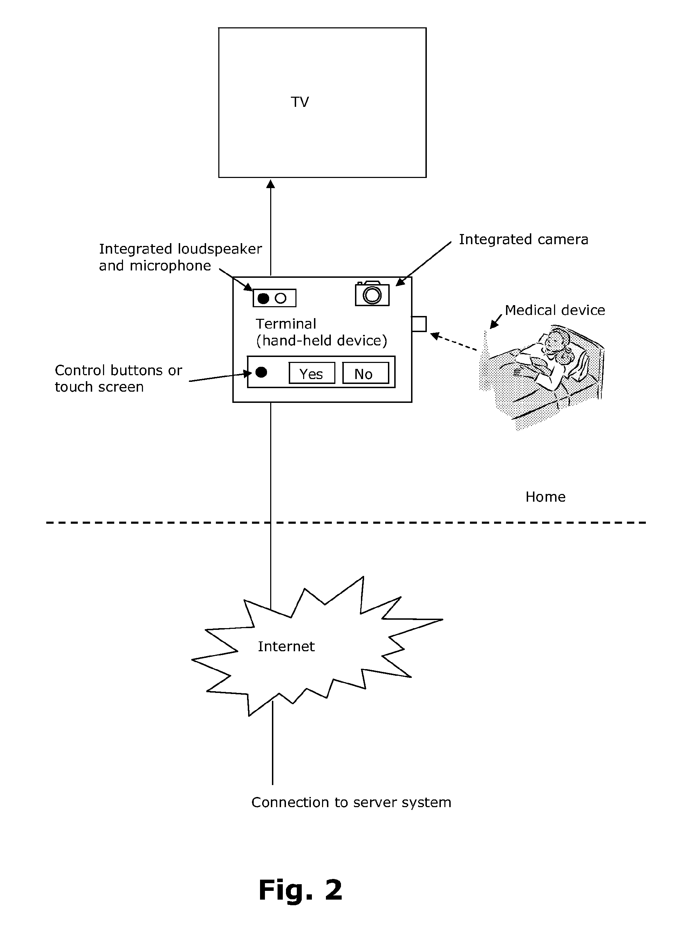

An embodiment of the home system as shown in Fig. 2 includes a terminal, such as a hand-held data interface device, which provides an interface allowing a patient to communicate data to and from the server system. The home system further includes a medical device for capturing health data related to a health condition of the patient. The medical device communicates with the terminal via a wired or wireless interconnection. The terminal may include an integrated monitor (not shown), or it may be connected to an external television apparatus as shown in Fig. 2. A camera is integrated in the terminal, as well as a loudspeaker and a microphone. The terminal further comprises a control panel and/or a touch screen display allowing the patient to switch the terminal on, initiate data transmission to the server and healthcare facility, and to input answers to pre-programmed or pre-packed questions stored in the terminal. The terminal (hand-held data interface device) is programmable from a remote site via the wide area network, e.g. Internet.

The terminal may optionally be provided in the form of a non-mobile device. A remote control unit (not shown) may be provided in order to allow the patient to interface with the terminal when at a distance therefrom.

In order to enhance data transmission security, the terminal may include hardware- or software-embedded encryption and/or authentication insignia for appropriately identifying the terminal to the server system and for encrypting data to the server system and decrypting data from the server system.

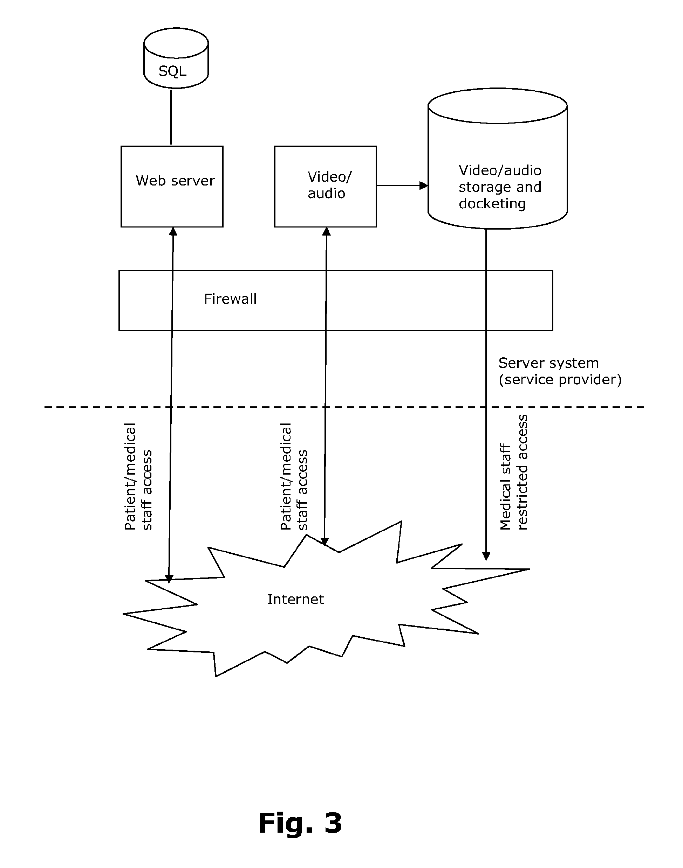

Fig. 3 illustrates an embodiment of the server system comprising a firewall, a web server, an SQL database, a video/audio server, and a video/audio storage and docketing database. Communication of plain health data is passed via the web server and stored in the SQL database. Patients as well as medical staff are given access to the web server, subject to appropriate user authentication and/or encryption. The SQL database stores and dockets health data captured by the medical device and communicated to the server system by the terminal of the home system (see Fig. 1), as well as patient responses to questions posed by the terminal. Videoconference sessions are passed through the video/audio server, which allows access by patients as well as medical staff. The video/audio server stores and dockets videoconference sequences, which may subsequently be retrieved by medical staff only.

It will be appreciated that the system, data interface device and methods of the present invention is suitable in the monitoring and treatment of a variety of diseases and physiological conditions requiring interaction between the patient and medical staff. For example, the invention is useful with regard to chronic diseases, such as diabetes, chronic obstructive pulmonary disease, heart diseases, diseases in the gastrointestinal system, cancer, organ diseases, including kidney diseases, aids, sclerosis, and arthritis. Other applications are also contemplated, e.g. monitoring of the physiological of athletes, or monitoring of psychological and psychiatric conditions.

Generally, the server system as applied in embodiments of the present invention may be configured to store a copy of the health care data passed therethrough, including data captured by the medical device as well as user-input, e.g. response to standardised questions or typed or spoken user input. Further, the server system may be configured to store a copy of videoconference sequences passed therethrough.

The data interface device, which is preferably a hand-held device, may comprise:
- data input structure for receiving the health data communicated by the at least one medical device;
- a network interface for connecting the data interface device to the wide area network;
- a user interface configured to receive a user input.

The data input structure as well as the network interface may include wireless or wired communication hardware and software. The user interface may include speech recognition circuitry, a keyboard, touch screen or any other type of interface. The user interface may be configured to limit user input to pre-defined response options to pre-programmed or pre-packed questions passed to the user, such as:
- How well did you sleep last night?
   Response option A: well
   Response option B: not well
   Response option C: bad
- Have you suffered from breathing difficulties within the last 24 hours?
   Response option A: no, not at all
   Response option B: yes, a little
   Response option C: yes, very much

The user input, notably the responses to the predefined questions, may be passed to the healthcare facility system and/or to the server system before videoconference contact is established between the patient and said medical staff, in order to allow the medical staff to examine the response before initiating videoconference contact with the patient.

The data interface device, i.e. user terminal, may comprise audio and video structure configured to enable videoconferencing by means of the data interface device, the audio and video structure including a monitor, a loudspeaker, and a microphone integrated in a housing of the data interface device. As an alternative to a built-in monitor, the terminal may be configured to communicate video data to an external monitor, e.g. a television apparatus.

As it will be apparent from the above description, the server system may comprise:
- an audio/video server for communicating audio and video data between the home system and the healthcare facility system;
- a web server for communicating other data between the home system and the healthcare facility system;
- an audio/video database server connected to the audio/video server and comprising a database for storing videoconference sequences;
- a non-video database server connected to the web server and comprising a database for storing the health data.

## Claims

1. A home health system comprising:
**(a)** a home system comprising at least one medical device for capturing health data related to a health condition of a patient, the home system further comprising a data interface device configured to receive the health data from the at least one medical device and to pass the health data into a wide area network;
**(b)** a healthcare facility system at a site remote from the home system, the healthcare facility system being configured to receive said health data communicated from the data interface device of the home system via the wide area network;
**(c)** videoconferencing equipment connected to or integrated with the home system and the healthcare facility system, the videoconferencing being configured to enable videoconferencing between the patient and medical staff at the medical health care facility via the wide area network;
**(d)** a server system at a site remote from the home system and remote from the healthcare facility system;
wherein the home system, the health care facility system, the videoconferencing equipment and the server system are configured such that all video data and health care data communicated between the home system and the healthcare facility system are passed through the server system.

2. A home health system according to claim 1, wherein the server system is configured to store a copy of the health care data passed therethrough.

3. A home health system according to claim 1 or 2, wherein the server system is configured to store a copy of videoconference sequences passed therethrough.

4. A home health system according to any of claims 1-3, wherein the home system comprises a hand-held data interface device, comprising:
- data input structure for receiving the health data communicated by the at least one medical device;
- a network interface for connecting the data interface device to the wide area network;
- a user interface configured to receive a user input.

5. A home health system according to claim 4, wherein the user interface is configured to limit user input to pre-defined response options to pre-programmed or pre-packed questions passed to the user.

6. A home health system according to claim 4 or 5, wherein the hand-held data interface device comprises audio and video structure configured to enable videoconferencing by means of the data interface device, the audio and video structure including a monitor, a loudspeaker, and a microphone integrated in a housing of the data interface device.

7. A home health system according to claim 4 or 5, wherein the hand-held data interface device comprises an audio/video interface for enabling videoconferencing via an external monitor.

8. A home health system according to any of the preceding claims, wherein the server system comprises:
- an audio/video server for communicating audio and video data between the home system and the healthcare facility system;
- a web server for communicating other data between the home system and the healthcare facility system;
- an audio/video database server connected to the audio/video server and comprising a database for storing videoconference sequences;
- a non-video database server connected to the web server and comprising a database for storing the health data.

9. A data interface device for use in a home health system according to any of claims 1-8, comprising:
- data input structure for receiving health data communicated by an external device;
- a network interface for connecting the data interface device to a wide area network;
- audio and video structure configured to enable videoconferencing by means of the data interface device, the audio and video structure including a monitor, a loudspeaker, and a microphone integrated in a housing of the data interface device;
- a user interface configured to receive a user input, the user input being limited to pre-defined response options to pre-programmed or pre-packed questions passed to the user.

10. A device according to claim 9, wherein the data input structure comprises a wireless communication interface.

11. A device according to claim 9 or 10, wherein the pre-programmed or pre-packed questions are programmable or editable from a remote location via the wide area network.

12. A method of monitoring a health condition of a chronically ill patient, comprising the steps of:
- providing a home system in the patient's home, the home system comprising at least one medical device for capturing health data related to said health condition;
- passing the health data into a wide area network;
- providing a healthcare facility system at a site remote from the home system, and receiving said health data at the healthcare facility via the wide area network;
- enabling a videoconference between the patient and medical staff at the medical health care facility via the wide area network;
- providing a server system at a site remote from the home system and remote from the healthcare facility system;
the method further comprising the step of passing all video data and health care data communicated between the home system and the healthcare facility system through the server system.

13. A method according to claim 12, wherein said health condition relates to a condition or state of chronic obstructive pulmonary disease, notably to the patient's lung function.

14. A method according to claim 12 or 13, wherein the home system comprises a user interface configured to receive a user input, and wherein the user input is passed to the server system before videoconference contact is established between the patient and said medical staff.

15. A method according to any of claims 12-14, wherein the user input is limited to pre-defined response options to pre-programmed or pre-packed questions passed to the user by the home system.
